# EUROPEAN PATENT APPLICATION

(11) **EP 4 191 638 A1**
(43) Date of publication of application: **07.06.2023**
(21) Application number: 21848553.0
(22) Date of filing: 27.07.2021
(51) Int. Cl.: H01L 21/00

(54) **SURFACE LINKER OF SEMICONDUCTOR CHIP, PREPARATION METHOD THEREFOR AND APPLICATION THEREOF**

(30) Priority: 27.07.2020 CN 202010730125
(71) Applicant: Nanjing GenScript Biotech Co., Ltd., Nanjing, Jiangsu 211100 (CN)
(72) Inventor: HU, Feichi, Nanjing, Jiangsu 211100 (CN); LIU, Chunyan, Nanjing, Jiangsu (CN); WU, Cheng-Hsien, Nanjing, Jiangsu 211100 (CN); LIN, Yonglong, Hsinchu City Taiwan (TW)
(74) Representative: Pharma Patents International AG
(86) International application number: PCT/CN2021/108534
(87) International publication number: WO 2022/022480

(57) **Abstract**

The present invention relates to the field of biochips, and provides a surface linker for a semiconductor chip, a preparation method therefor and an application thereof. The chip surface linker reacts with a chip surface by means of using silanized molecules as a solute and toluene as a solvent so as to form bonding molecules connected to the chip surface, and is prepared by reacting with functionalized molecules to modify a hydroxyl group and an ester group. The chip surface linker obtained by the present invention may be stably bonded to the chip surface, is stable under acidic and alkaline conditions, has good electrical conductivity, electrical stability and resistance to organic solvents required for nucleic acid synthesis, and is extremely advantageous for subsequent nucleic acid synthesis and other applications.

## Description

### TECHNICAL FIELD

The present invention relates to the technical field of biochip preparation, and particularly to a surface linker for nucleic acid synthesis on a TiN chip, and a preparation method and use thereof.

### BACKGROUND

A stable linker is very important for nucleic acid synthesis on a semiconductor chip. The linker can be used for in situ synthesis or pre-preparation of a large number of DNA probes on the surface, and can also be firmly linked to nucleic acids for subsequent in situ hybridization and other applications. Through the detection and analysis of hybridization signals of a sample, genetic information (information about genetic sequences and expressions) of the sample is obtained. Nucleic acid synthesis on semiconductor chips can play an important role in the fields such as disease diagnosis, drug screening and new drug development, agriculture and environmental research. Nucleic acid synthesis on a precious metal is mostly used in the market. A variety of methods, including multiple linkers and multiple nucleic acid synthesis methods, may be used. However, stable linkers for nucleic acid synthesis on semiconductor chips are rare. Since semiconductors are inexpensive and semiconductor chips are easy to prepare and more suitable for industrial mass production, it is important to develop a semiconductor chip linker to promote the application of semiconductor chips. At present, mostly, after simple modification of a molecule on a semiconductor, a polymer is linked or other materials are grafted. For example, Y. Shacham-Diamand studied the use of a chemical method to modify a silanized molecule on TiN and then plate a Co(W,P) material [1]. Xuan Tuan Le used a diazonium salt method to modify the TiN surface and then uses it for plating a polymer [2]. The problem with this method is that it requires electric modification, which is not conducive to large-scale mass production. Another method is to use the direct adsorption method to adsorb a small molecule on the surface of TiN [3]. The disadvantage of this method is that the adhesion is not strong and is sensitive to water, and especially the molecule will fall off in later use, making it difficult to further develop the required chip-based diagnostic products.

### SUMMARY

In view of this, the present invention provides a stable linker for nucleic acid synthesis on a semiconductor TiN chip. The main method is carrying out a silanization reaction on a clean TiN surface for surface amination, and then modifying the surface with a functional molecule containing a hydroxyl/hydroxyl protecting group and an ester group by carboxylamino reaction, so that subsequent in-situ synthesis of nucleic acids on the chip can be achieved.

According to an aspect, the present invention provides a chip surface linker, wherein the linker is prepared by using a silanized molecule to react with a surface of a chip to form a bonding molecule linked to the surface of the chip, and further causing the bonding molecule to react with a functional molecule and be modified with a hydroxyl group and an ester group. Specifically, the present invention provides a chip surface linker, where the linker is prepared by using a silanized molecule to react with a surface of a chip in a suitable solvent to form a bonding molecule linked to the surface of the chip, and causing the bonding molecule to react with a functional molecule and be modified with a hydroxyl group and an ester group. The hydroxyl group may be a free hydroxyl group or a protected hydroxyl group.

In some embodiments, the silanized molecule is selected from one or more of 3-aminopropyltriethoxysilane, dodecyltrimethoxysilane, vinyltriethoxysilane, bis(γ-trimethylsilylpropyl)amine, or γ-(2,3-epoxypropoxy)propyltrimethoxysilane, and is preferably 3-aminopropyltriethoxysilane.

In some embodiments, the silanized molecule is reacted with the surface of the chip in a suitable solvent. In some embodiments, the solvent is selected from one or more of toluene, ethanol or methanol, and is preferably toluene.

In some embodiments, a volume ratio of the solvent to the silanized molecule is 2:1 to 40:1, preferably 20:1 to 3 5:1, and more preferably 30: 1. The volume ratio of the solvent to the silanized molecule may be selected from the group consisting of 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, or 40:1.

In some embodiments, the silanized molecule is reacted with the surface of the chip at a temperature of 50°C to 100°C, preferably 60°C to 80°C, and more preferably 65°C. The specific reaction temperature of the reaction may be selected from 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, or 100°C. In some embodiments, the silanized molecule is reacted with the surface of the chip for preferably 1 to 6 h, more preferably 2 to 5 h, and most preferably 4 h. Other preferred reaction times are 1 h, 1.5 h, 2 h, 2.5 h, 3 h, 3.5 h, 4 h, 4.5 h, 5 h, 5.5 h, or 6 h. In some embodiments, the silanized molecule is reacted with the surface of the chip at 50°C to 100°C for 1 to 6 h. In some embodiments, the silanized molecule is reacted with the surface of the chip at 60°C to 80°C for 2 to 5 h. In a specific embodiment, the silanized molecule is reacted with the surface of the chip in a sealed reactor at 65°C for 4 h.

In some embodiments, the functional molecule is a hydroxyl-containing compound containing a long carbon chain and an ester group. In some other embodiments, the functional molecule is selected from one or more of a succinic anhydride-modified base monomer, hydroxyethyl methacrylate, a succinic acid-modified base monomer, or an oxalic acid-modified base monomer, and is preferably a succinic anhydride-modified base monomer. In some embodiments, the base monomer moiety of the functional molecule is selected from one or more of an adenine nucleoside, a guanine nucleoside, a cytosine nucleoside, a thymine nucleoside, or an uracil nucleoside. In a specific embodiment, the functional molecule is selected from one or more of a succinic anhydride-modified adenine nucleoside, guanine nucleoside, cytosine nucleoside, thymine nucleoside, or uracil nucleoside.

In some embodiments, before the bonding molecule is reacted with and modified by the functional molecule, a step of curing the bonding molecule linked to the surface of the chip under a suitable condition is further included.

In some embodiments, the curing condition is placing in a drying device at 60°C to 100°C for 1 to 6 h, and the curing/placement temperature is preferably 75°C to 95°C, and most preferably 90°C. Other preferred curing/placement temperatures are 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C or 95°C. The curing/placement time is preferably 1 to 4 h, most preferably 1 h. Other preferred placement times are 1 h, 1.5 h, 2 h, 2.5 h, 3 h, 3.5 h, or 4 h. In some specific embodiments, the curing condition is placing in a drying device at 90°C for 1 h.

In some embodiments, the chip is a semiconductor chip. In some specific embodiments, the surface of the semiconductor chip includes TiN (titanium nitride) or TiW (titanium tungsten), preferably TiN.

According to another aspect, the present invention further provides a method for preparing a chip surface linker, including the following steps:
step 1: mixing a silanized molecule with a suitable solvent in a volume ratio to obtain a mixed solution;
step 2: contacting and reacting a chip with the mixed solution in the step 1 to form a bonding molecule linked to a surface of the chip; and
step 3: contacting and reacting the surface of the chip linked to the bonding molecule after the reaction with a functional molecule to modify same with a hydroxyl group and an ester group.

In some embodiments, the silanized molecule in the step 1 is selected from one or more of 3-aminopropyltriethoxysilane, dodecyltrimethoxysilane, vinyltriethoxysilane, bis(γ-trimethylsilylpropyl)amine, or γ-(2,3-epoxypropoxy)propyltrimethoxysilane, and is preferably 3-aminopropyltriethoxysilane.

In some embodiments, the solvent in the step 1 is selected from one or more of toluene, ethanol or methanol, and is preferably toluene.

In some embodiments, a volume ratio of the solvent to the silanized molecule in the step 1 is 2:1 to 40:1, preferably 20:1 to 35:1, and more preferably 30:1. Other preferred volume ratios of the solvent to the silanized molecule are 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, or 40:1.

In some embodiments, in the step 2, the silanized molecule is reacted with the surface of the chip at 50°C to 100°C for 1 to 6 h. In some embodiments, the silanized molecule is reacted with the surface of the chip at 60°C to 80°C for 2 to 5 h. In a specific embodiment, the silanized molecule is reacted with the surface of the chip in a sealed reactor at 65°C for 4 h.

In some embodiments, the functional molecule is a hydroxyl-containing compound containing a long carbon chain and an ester group. In some other embodiments, the functional molecule is selected from one or more of a succinic anhydride-modified base monomer, hydroxyethyl methacrylate, a succinic acid-modified base monomer, or an oxalic acid-modified base monomer, and is preferably a succinic anhydride-modified base monomer. In some embodiments, the base monomer moiety of the functional molecule is selected from one or more of an adenine nucleoside, a guanine nucleoside, a cytosine nucleoside, a thymine nucleoside, or an uracil nucleoside. In a specific embodiment, the functional molecule is selected from one or more of a succinic anhydride-modified adenine nucleoside, guanine nucleoside, cytosine nucleoside, thymine nucleoside, or uracil nucleoside.

In some embodiments, before the step 3, the method further includes a step of curing the bonding molecule linked to the surface of the chip under a suitable condition.

In some embodiments, the curing condition is placing in a drying device at 60°C to 100°C for 1 to 6 h, preferably placing in a drying device at 75°C to 95°C for 1 to 3 h, and most preferably placing in a drying device at 90°C for 1 h. In the curing condition, the placement temperature may be selected from 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, or 95°C. The curing/placement time is preferably 1 to 4 h, most preferably 1 h. Other preferred placement times may be selected from 1 h, 1.5 h, 2 h, 2.5 h, 3 h, 3.5 h, or 4 h. In some specific embodiments, the curing condition is placing in a drying device at 90°C for 1 h.

In some embodiments, the chip is a semiconductor chip. In some specific embodiments, the surface of the semiconductor chip includes TiN or TiW, preferably TiN.

The present invention also provides use of the above-described chip surface linker in nucleic acid synthesis or in the preparation of a chip kit.

The present invention provides a chip surface linker, particularly a semiconductor chip linker. The linker is prepared by using a silanized molecule to react with a surface of a chip in a suitable solvent to form a bonding molecule linked to the surface of the chip, and causing the bonding molecule to react with a functional molecule and be modified with a hydroxyl group and an ester group.

The silanized molecule in the present invention may be 3-aminopropyltriethoxysilane, dodecyltrimethoxysilane, vinyltriethoxysilane, bis(γ-trimethylsilylpropyl)amine, or γ-(2,3-epoxypropoxy)propyltrimethoxysilane. In some embodiments of the present invention, the silanized molecule is 3-aminopropyltriethoxysilane. The bonding molecule in the present invention is a group that is covalently attached to the surface of the chip after the silanized molecule reacts with the surface of the chip. In some embodiments of the present invention, the silanized molecule APTES (3-aminopropyltriethoxysilane) is dissolved in a suitable solvent such as toluene (where the volume ratio of the solvent to the silanized molecule is 2:1 to 40: 1, preferably 30:1) and is reacted with the surface of the TiN-containing semiconductor chip at a high temperature of 50°C to 100°C (preferably 65°C) for 1 to 6 h (preferably 4 h) to form a bonding molecule on the surface of the chip.

In some embodiments of the present invention, before the bonding molecule is reacted with and modified by the functional molecule, a step of curing the bonding molecule linked to the surface of the chip under a suitable condition is further included. In some embodiments of the present invention, the curing condition is placing the chip having reacted with the silanized molecule in an oven at 60°C to 100°C. for 1 to 6 h, preferably in an oven at 90°C for 1 h.

The functional molecule in the present invention is a hydroxyl-containing compound containing a long carbon chain and an ester group, and may be a succinic anhydride-modified base monomer, hydroxyethyl methacrylate, a succinic acid-modified base monomer, or an oxalic acid-modified base monomer. In some embodiments of the present invention, the functional molecule is a mixture of one or more of a succinic anhydride-modified adenine nucleoside, guanine nucleoside, cytosine nucleoside, or thymine nucleoside. The long carbon chain in the present invention refers to a long chain structure containing a plurality of carbon-carbon covalent bonds in a molecule, and may contain 2 or more carbon atoms, preferably 2 to 4 carbon atoms. The longer the molecular structure of the hydroxyl-containing compound containing the long carbon chain and the ester group, the smaller the spatial impediment during subsequent nucleic acid synthesis, facilitating synthesis. In some embodiments of the present invention, a succinic anhydride-modified adenine nucleoside, a succinic anhydride-modified guanine nucleoside, a succinic anhydride-modified cytosine nucleoside, or succinic anhydride-modified thymine nucleoside solution is used alone. In some embodiments, the functional molecule may also be selected from a group that reacts with an amino group to release a hydroxyl in a succinic anhydride-modified adenosine nucleoside, guanosine nucleoside, cytidine nucleoside, thymidine nucleoside or uracil nucleoside; in hydroxyethyl methacrylate; in a succinic acid-modified adenosine nucleoside, guanosine nucleoside, cytidine nucleoside, thymidine nucleoside or uracil nucleoside; or in an oxalate-modified adenosine nucleoside, guanosine nucleoside, cytidine nucleoside, thymidine nucleoside or uracil nucleoside. In the present invention, the functional molecule is modified on the bonding molecule, and the formed functional molecular group contains a hydroxyl group and an ester group, which facilitates the subsequent synthesis and cleavage of electrically-promoted DNA on the chip.

The hydroxyl group for modification through the reaction with the functional molecule in the present invention may be a hydroxyl group, i.e., -OH, or may be a protected hydroxyl group, e.g., -ODMT. A hydroxyl protecting group is a hydroxyl group protected by a commonly used protecting group. In some embodiments of the present invention, the hydroxyl group for modification through the reaction with the functional molecule is a hydroxyl group. In some other embodiments, the hydroxyl group for modification through the reaction with the functional molecule is ODMT.

The chip surface linker of the present invention is stably bonded to the surface of the chip through a covalent bond, and can be used for electro-assisted nucleic acid synthesis on a chip and other applications. The semiconductor chip is a commonly seen chip including a semiconductor material in the art. The semiconductor chip in the present invention may be selected from an ordinary chip-shaped semiconductor chip having a surface containing TiN or TiW. In a specific embodiment of the present invention, the chip is a semiconductor chip that can be applied to a CustomArray chip synthesizer. A step of cleaning the chip is included before the reaction of linking the chip. In the present invention, the step of cleaning the chip may include rinsing with water, alcohol, and water in sequence, followed by drying, to obtain a dry and clean chip surface. The water is generally distilled water, the alcohol is ethanol or methanol, and mainly pollutants such as other impurity metal dust, inorganic particles and organic small molecules are removed.

The present invention also provides a method for preparing a chip surface linker, including the following steps: mixing a silanized molecule with a suitable solvent in a volume ratio to obtain a mixed solution; contacting and reacting a cleaned chip with the mixed solution to form a bonding molecule linked to a surface of the chip; and contacting and reacting the surface of the chip after the reaction with a functional molecule to modify same with a hydroxyl group and an ester group, to form a linker. In some embodiments, before the bonding molecule is reacted with and modified by the functional molecule, a step of curing the bonding molecule linked to the surface of the chip under a suitable condition is further included.

In some embodiments of the present invention, toluene as the solvent and the silanized molecule are mixed in a volume ratio of 2:1 to 40:1, preferably 30:1, to prepare the mixed solution. The silanized molecule may be 3-aminopropyltriethoxysilane, dodecyltrimethoxysilane, vinyltriethoxysilane, bis(γ-trimethylsilylpropyl)amine, or γ-(2,3-epoxypropoxy)propyltrimethoxysilane, and is preferably 3-aminopropyltriethoxysilane. The cleaned chip is reacted with the mixed solution at 50°C to 100°C, preferably at 65°C for 1 to 6 h, preferably for 4 h. The specific reaction is as follows:

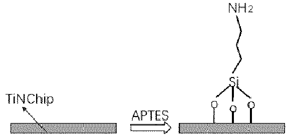

After the reaction, the chip is taken out, then rinsed with water and alcohol in sequence, and finally rinsed with water and blow dried.

The chip after the reaction was treated under a suitable condition to cure the bonding molecule linked to the surface of the chip. The curing condition is placing in a drying device at 60°C to 100°C for 1 to 6 h. The curing/placement temperature is preferably 75°C to 95°C, and most preferably 90°C. The curing/placement time is preferably 1 to 4 h, most preferably 1 h. The temperature for curing the chip in the present invention is generally higher than the temperature for linking the silanized molecule to the surface of the chip.

The cured chip is then modified by the functional molecule. The functional molecule is a hydroxyl-containing compound containing a long carbon chain and an ester group. The hydroxyl-containing compound includes, but is not limited to, a succinic anhydride-modified base monomer, hydroxyethyl methacrylate, or an oxalic acid-modified base monomer. In some embodiments of the present invention, a solution of a succinic anhydride-modified adenine nucleoside, guanine nucleoside, cytosine nucleoside, thymine nucleoside, or uracil nucleoside is used alone. In some specific embodiments of the present invention, a mixture of two or more of a succinic anhydride-modified adenine nucleoside, guanine nucleoside, cytosine nucleoside, thymine nucleoside, or uracil nucleoside is used. The functional molecule contains an ester group, which can be used for DNA synthesis and cleavage. The ester group is the cleavage site for cleaving the DNA synthesized on the chip to form a free solution oligo pool. The hydroxyl group contained in the functional molecule is the starting site of DNA synthesis. In some embodiments of the present invention, the hydroxyl group provided by the base monomer moiety is used in DNA synthesis. In addition, the hydroxyl molecular solution may further contain an activator for functional modification, such as NHS (N,N-hydroxysuccinimide) or EDC (1-(3-dimethylaminopropyl)-3-ethylcarbodiimide hydrochloride).

The present invention also provides use of the above-described chip surface linker in DNA synthesis or in the preparation of a chip kit. The chip surface linker of the present invention includes the following applications: for DNA synthesis on a chip; for chip-based disease biomarker detection; for development of a chip kit for POCT; and for development of a high throughput chip screening kit.

The chip surface linker in the present invention can be used to synthesize DNA. After being hybridized to an oligonucleotide (such as a DNA primer) and then subjected to alkaline and thermal TE treatment, the synthesized DNA may further hybridized to an oligonucleotide (such as a DNA primer). After the DNA synthesized by the chip surface linker of the present invention is subjected to a number of nucleic acid hybridization, elution, and hybridization cycles, the chip surface linker can still maintain good stability. The chip surface linker prepared in the embodiments of the present invention has good electrical conductivity and electrification stability, and can be used for electro-assisted synthesis of nucleic acid molecules such as DNA. If the linker does not have this property, DNA synthesis will be affected. The chip surface linker of the present invention is resistant to organic solvents required for DNA synthesis (including 4,5-dicyanoimidazole, trichloroacetic acid, acetic anhydride, 1-methylimidazole, iodine solution, etc.), resistant to acids and bases, and resistant to ammonia. Otherwise, DNA cannot be synthesized. When the chip linker is used for chip hybridization detection, the present invention can solve the problems that existing linkers are sensitive to the synthesis environment of water, acids and bases, and organic solvents, and are thermally unstable, so the chip can be utilized repeatedly.

The term "chip" refers to a solid support formed from an inorganic material such as a semiconductor or a metal such as gold, silver, platinum, etc., with a surface having a microarray formed of specific sites, typically arranged in rows and columns, where the sites can be used for certain types of chemical or biochemical analysis, synthesis, or methods. These sites on the microarray are typically less than 100 microns. In some embodiments of the present invention, the chip is a TiN-containing semiconductor chip.

The term "bonding molecule" means a chemical molecule located at the end of the linker, which can be covalently attached at one end to a solid surface (such as a TiN-containing semiconductor chip), and has a reactive group at the other end, where the reactive group is or can be attached to an associated chemical substance, such as a small molecule, oligomer, or polymer. The reactive group can be attached to a functional molecule in the present invention. The bonding molecule is already bound to the solid surface and/or the reactive group of the bonding molecule is already attached to an associated chemical substance. The reactive group of the bonding molecule may be attached to a protecting group, where the protecting group may be removed by a chemical or electrochemical method.

The term "functional molecule" means a chemical molecule located at the end portion of the linker, which can be covalently attached at one end to the bonding molecule, and has a reactive group at the other end, where the reactive group is or can be attached to an associated chemical substance, such as a small molecule, oligomer, or polymer. The reactive group can be attached to a deoxyribonucleotide molecule in the present invention. The functional molecule may be already bound to the bonding molecule and/or the reactive group of the functional molecule may be already attached to an associated chemical substance. The reactive group of the functional molecule may be attached to a protecting group, where the protecting group may be removed by a chemical or electrochemical method. The functional molecule in the present invention may be a succinic anhydride-modified adenosine nucleoside, guanosine nucleoside, cytidine nucleoside, thymidine nucleoside or uracil nucleoside; hydroxyethyl methacrylate; a succinic acid-modified adenosine nucleoside, guanosine nucleoside, cytidine nucleoside, thymidine nucleoside or uracil nucleoside; or an oxalate-modified adenosine nucleoside, guanosine nucleoside, cytidine nucleoside, thymidine nucleoside or uracil nucleoside.

The term "base monomer" means a molecule that is capable of polymerizing to form a constituent unit of a basic structure of a macromolecule, e.g., an oligomer, a co-oligomer, a polymer, or a copolymer. Examples of monomers include A, C, T, G, adenine nucleosides, guanine nucleosides, cytosine nucleosides, thymine nucleosides, uracil nucleosides, adenylate, guanylate, cytidine, uridine, amino acids, and other compounds.

The term "oligomer" means a molecule having an intermediate relative molecular mass, the structure of which essentially includes a small number of units that are actually or conceptually derived from a lower relative molecular mass molecule. A molecule may be considered to have a medium relative molecular mass if its properties differ significantly after removal of one or more units of the molecule. If a portion or the whole of the molecule has a medium relative molecular mass and substantially includes a small number of units that are actually or conceptually derived from a lower relative molecular mass molecule, the molecule may be described as oligomeric, or as an oligomer used as an adjective. Oligomers are typically composed of monomers.

### Beneficial effects

The present invention provides a stable linker for nucleic acid synthesis on a surface of a semiconductor TiN chip, and a preparation method and use thereof. In the present invention, the silanized molecule serving as the solute and toluene serving as the solvent are reacted with the surface of the TiN chip at a high temperature for a certain period of time and then cured at a higher temperature, and finally, the silanized molecule is modified by a functional molecule containing an ester group and a hydroxyl group, thus completing the preparation of this stable linker. This linker has good stability and is resistant to organic solvents, strong acids and strong bases. It can be used for in-situ nucleic acid synthesis on the surface of the TiN chip, and can be further used for in-situ hybridization on the surface of the chip, providing the possibility and laying a foundation for the development of related chip-based diagnostic products.

### BRIEF DESCRIPTION OF THE DRAWINGS

FIG. 1 is a flowchart showing preparation of a TiN chip linker;
FIG. 2 is a diagram of a chromogenic test for feasibility of the TiN chip linker;
FIG. 3 is a diagram of a stability test against organic solvents of the TiN chip linker;
FIG. 4 is a diagram of a stability test against ammonia of the TiN chip linker;
FIG. 5 is a diagram of a stability test against acids and bases of the TiN chip linker; and
FIG. 6 is a diagram showing the results of application of the TiN chip linker in DNA synthesis and hybridization.

### DETAILED DESCRIPTION

The technical solutions in the embodiments of the present invention are described clearly and completely below. Apparently, the embodiments described are merely some embodiments, rather than all of the embodiments of the present application. All other embodiments obtained by those of ordinary skill in the art without creative efforts based on the embodiments of the present invention shall fall within the protection scope of the present invention.

In order to provide a further understanding of the present invention, the chip surface linker and the preparation method and use thereof provided in the present invention are described in detail below in conjunction with examples. However, it should be understood that these examples are implemented on the premise of the technical solutions of the present invention, detailed implementations and specific operating procedures are set forth only for the purpose of further explaining the features and advantages of the present invention rather than limiting the claims of the present invention, and the scope of protection of the present invention is also not limited to the following examples.

### Example 1: Linker preparation test

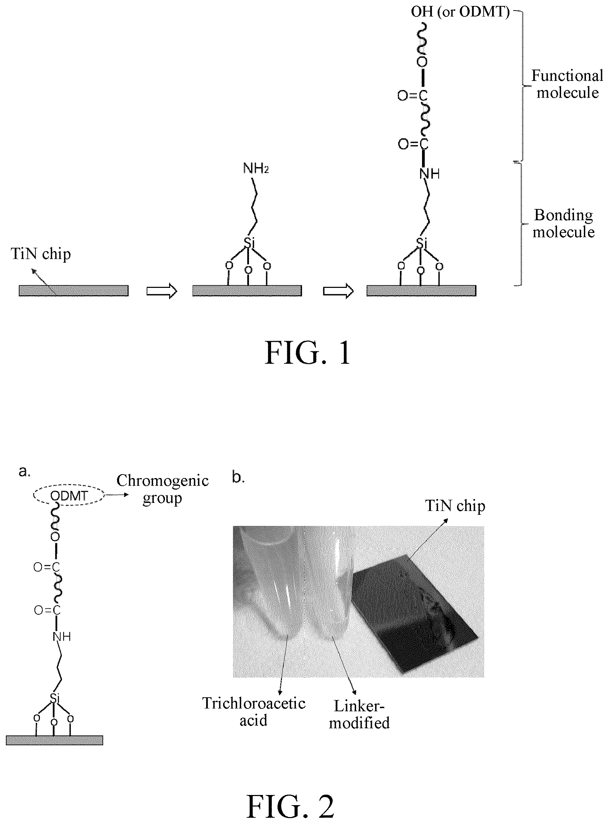

Specific experimental steps for linker preparation are as follows.
1. A TiN chip (from an IC factory) was rinsed 5 times with distilled water, 5 times with ethanol, 5 times with methanol, 5 times with distilled water, and blow dried with nitrogen.
2. A mixture of toluene and APTES (3-aminopropyltriethoxysilane) (30:1, volume ratio) was formulated. The TiN chip was immersed in this mixture, and reacted at 65°C for 4 h after sealing. The specific reaction is as follows: 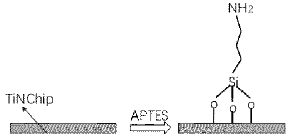
3. The chip was taken out, placed in an oven at 90°C, cured at high temperature for 1 h, then washed with ethanol 3 times, washed with distilled water 3 times, and blow dried with nitrogen.
4. The chip was immersed in a mixed solution of 10.8 mg of a succinic anhydride-modified base monomer (where the base monomer was adenine nucleoside, thymine nucleoside, guanine nucleoside or cytosine nucleoside), 1.53 mg of NHS, and 7.64 mg of EDC dissolved in 100 µL of water), and allowed to stand for 8 h.
5. The chip was rinsed with ethanol, acetone, ethanol and distilled water 5 times in sequence, and blow dried with nitrogen to obtain this stable linker, as shown in FIG. 1. The linker includes a bonding molecule and a functional molecule, where the bonding molecule is a silanized molecule and the functional molecule is a molecule containing a hydroxyl (or protected hydroxyl) group and an ester group.

The linker is prepared on the surface of the TiN chip by the above method, rinsed with a large amount of ethanol, then rinsed with distilled water, and blow dried with nitrogen. 20 µL of trichloroacetic acid was added to the linker-modified chip surface. After 1 second, the trichloroacetic acid was quickly collected to a centrifuge tube. As shown in FIG. 2, a significant color change (slight red) was observed compared with the trichloroacetic acid stock solution. This is due to the chromogenic reaction of the ODMT group (4,4'-bimethoxytrityl, ODMT on the base molecule monomer) at the top of the linker with the trichloroacetic acid, indicating that the linker was successfully modified on the surface.

### Example 2: Linker stability test

### 2.1 Linker stability against organic solvents

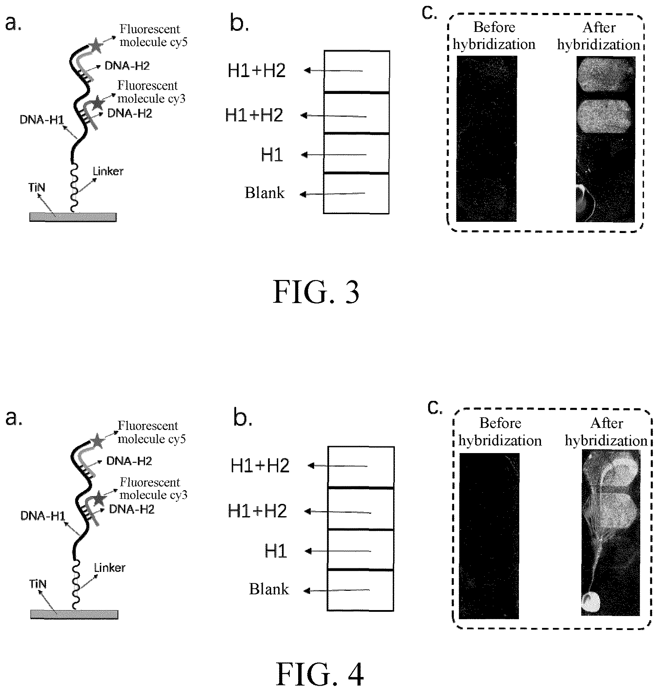

In order to test the stability of the linker against organic solvents, particularly the stability of the silanized molecule that react with the TiN surface, the silanized molecule was first reacted with the surface of the TiN chip according to the method of Example 1 (not including the functional molecule modification step). Then the chip was immersed in an organic solvent (including 4,5-dicyanoimidazole, trichloroacetic acid, acetic anhydride, 1-methylimidazole, and iodine solution, in a volume ratio of 1:1:1:1:1) used for synthesizing an oligonucleotide, and allowed to stand at room temperature for two days. Then, after rinsing with ethanol and distilled water, a probe DNA-H1 (2 µM, Nanjing GenScript Biotech Co., Ltd.) was linked to the silanized molecule through the reaction of carboxyl and amino groups, and then probes DNA-H2 modified with cy5 and cy3 fluorescent molecules (10 nM, Nanjing GenScript Biotech Co., Ltd.) were hybridized with the DNA-H1, as shown in FIG. 3a. As shown in FIG. 3b, the chip hybridization design was divided into four parts, where the upper two parts were hybridizations, and the lower two parts were for comparison. After hybridization, it was scanned under a chip scanner (CustomArray, GenePix 4000B) (532 nm/635 nm). It can be found that obvious fluorescence was only observed in the hybridized portion, as shown in FIG. 3c, which also indicates that the silanized molecule in the first modification step was still stable on the surface of the TiN chip after being soaked with organic solvents, and can be used for subsequent reactions and applications.

The sequence of the DNA-H1 probe was:
5'-ACACTCTTTCCCTACACGACGCTCTTCCGATCTTTTTT-NH2-3' (SEQ ID NO:1)

The sequence of the DNA-H2-cy5 probe was:
5'-TAGGGAAAGAGTGT-Cy5-3' (SEQ ID NO:2)

The sequence of the DNA-H2-cy3 probe was:
5'-AGATCGGAAGAGCG-Cy3-3' (SEQ ID NO:3)

### 2.2 Linker stability against ammonia

In order to test the stability of the linker against ammonia, particularly the stability of the silanized molecule that react with the TiN surface, the silanized molecule was first reacted with the TiN surface according to the method of Example 1 (not including the functional molecule modification step). Then the chip was immersed in 28% ammonia water, and allowed to stand at 65°C for 16 h. Then, after rinsing with ethanol and distilled water, a probe DNA-H1 (2 µM, Nanjing GenScript Biotech Co., Ltd., the sequence of which was the same as that in Example 2.1) was linked to the silanized molecule through the reaction of carboxyl and amino groups, and then probes DNA-H2 modified with cy5 and cy3 fluorescent molecules (10 nM, Nanjing GenScript Biotech Co., Ltd., the sequences of the two probes being the same as those of DNA-H2 in Example 2.1) were hybridized with the DNA-H1, as shown in FIG. 4a. As shown in FIG. 4b, the chip hybridization design was divided into four parts, where the upper two parts were hybridizations, and the lower two parts were for comparison. After hybridization, it was scanned under a chip scanner (532 nm/635 nm). It can be found that obvious fluorescence was only observed in the hybridized portion, as shown in FIG. 4c, which also indicates that the silanized molecule in the first modification step was still stable on the surface of the TiN chip after being soaked with the ammonia water, and can be used for subsequent reactions and applications.

### 2.3 Linker stability against acids and bases

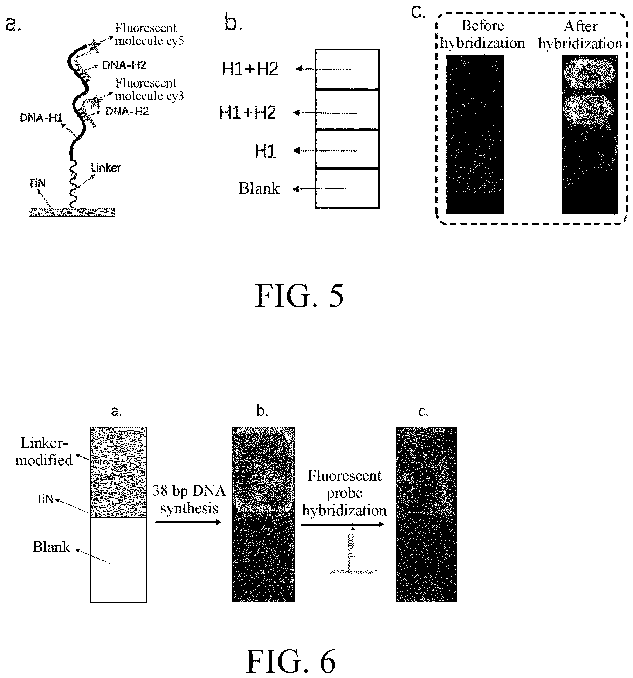

In order to test the stability of the linker against acids and bases, particularly the stability of the silanized molecule that react with the TiN surface, the silanized molecule was first reacted with the TiN surface according to the method of Example 1 (not including the functional molecule modification step). Then the chip was immersed in 0.5 M sodium hydroxide solution for 0.5 h, rinsed with distilled water, and then placed in 0.5 M hydrochloric acid solution for 0.5 h. Then, after rinsing with distilled water, a probe DNA-H1 (2 µM, Nanjing GenScript Biotech Co., Ltd., the sequence of which was the same as SEQ ID NO:1 in Example 2.1) was linked to the silanized molecule through the reaction of carboxyl and amino groups, and then probes DNA-H2 modified with cy5 and cy3 fluorescent molecules (10 nM, Nanjing GenScript Biotech Co., Ltd., the sequences of the two probes being the same as those of DNA-H2 in Example 2.1, i.e., SEQ ID NO:2 and SEQ ID NO:3) were hybridized with the DNA-H1, as shown in FIG. 5a. As shown in FIG. 5b, the chip hybridization design was divided into four parts, where the upper two parts were hybridizations, and the lower two parts were for comparison. After hybridization, it was scanned under a chip scanner. It can be found that obvious fluorescence was only observed in the hybridized portion, as shown in FIG. 5c, which also indicates that the silanized molecule in the first modification step was still stable on the surface of the TiN chip after being soaked with strong bases and strong acids, and can be used for subsequent reactions and applications.

### Example 3: Use of linker in DNA synthesis and hybridization

DNA was synthesized on a TiN chip modified with this linker. The TiN chip was divided into two parts, where the upper part is modified with the linker, and the lower part is not modified with the linker, as a blank control, as shown in FIG. 6a. This chip was placed on a CustomArray chip synthesizer. Reagents for DNA synthesis were injected one by one into the surface of the chip by using, for example, a method of column synthesis (see Reference [4]), to synthesize 38nt DNA (the sequence of which was the same as SEQ ID NO: 1 of the DNA-H1 in Example 2.1). After the synthesis, the chip was scanned on a chip scanner. As shown in FIG. 6b, the results show that a significant color change was observed in the linker-modified part and no color change was observed in the blank part, indicating that the DNA synthesis was successful. In order to verify whether the synthesized DNA is the desired DNA, a DNA probe modified with a cy3 fluorescent molecule (100 pM, Nanjing GenScript Biotech Co., Ltd., the sequence of the probe being the same as SEQ ID NO: 3 of the DNA-H2-cy3 in Example 2.1) was hybridized with the synthesized DNA. After hybridization, the chip was scanned on a chip scanner. As shown in FIG. 6c, the results show that a significant red light was observed, indicating that the hybridization was successful, and the synthesized sequence on the chip was the target sequence. This further proves that this linker can be used for in situ synthesis of DNA.

### References:

1. Malki, Maayan, et al. "Thin electroless Co (W, P) film growth on titanium-nitride layer modified by self-assembled monolayer." Surface and Coatings Technology 252 (2014): 1-7.
2. Zeb, Gul, et al. "On the chemical grafting of titanium nitride by diazonium chemistry." RSC Advances 5.62 (2015): 50298-50305.
3. Qiu, Guangyu, Siu Pang Ng, and Chi-Man Lawrence Wu. "Label-free surface plasmon resonance biosensing with titanium nitride thin film." Biosensors and Bioelectronics 106 (2018): 129-135.
4. Lönnberg H. Synthesis of oligonucleotides on a soluble support[J]. Beilstein journal of organic chemistry, 2017, 13(1): 1368-1387.

## Claims

1. A chip surface linker, wherein the linker is prepared by using a silanized molecule to react with a surface of a chip to form a bonding molecule linked to the surface of the chip, and further causing the bonding molecule to react with a functional molecule and be modified with a hydroxyl group and an ester group.

2. The chip surface linker according to claim 1, wherein the silanized molecule is selected from one or more of 3-aminopropyltriethoxysilane, dodecyltrimethoxysilane, vinyltriethoxysilane, bis(γ-trimethylsilylpropyl)amine, or γ-(2,3-epoxypropoxy)propyltrimethoxysilane, and is preferably 3-aminopropyltriethoxysilane.

3. The chip surface linker according to claim 1 or 2, wherein the silanized molecule is reacted with the surface of the chip in a suitable solvent, and the solvent is selected from one or more of toluene, ethanol or methanol, and is preferably toluene.

4. The chip surface linker according to claim 3, wherein a volume ratio of the solvent to the silanized molecule is 2:1 to 40:1, preferably 30:1.

5. The chip surface linker according to any one of claims 1 to 3, wherein the silanized molecule is reacted with the surface of the chip at a temperature of 50°C to 100°C, preferably 65°C.

6. The chip surface linker according to any one of claims 1 to 5, wherein the silanized molecule is reacted with the surface of the chip for 1 to 6 h, preferably 4 h.

7. The chip surface linker according to any one of claims 1 to 6, wherein the functional molecule is a hydroxyl-containing compound containing a long carbon chain and an ester group.

8. The chip surface linker according to claim 7, wherein the functional molecule is selected from one or more of a succinic anhydride-modified base monomer, hydroxyethyl methacrylate, a succinic acid-modified base monomer, or an oxalic acid-modified base monomer, and is preferably a succinic anhydride-modified base monomer, wherein the base monomer moiety is selected from one or more of an adenine nucleoside, a guanine nucleoside, a cytosine nucleoside, a thymine nucleoside, or an uracil nucleoside.

9. The chip surface linker according to any one of claims 1 to 8, wherein before the bonding molecule is reacted with and modified by the functional molecule, a step of curing the bonding molecule linked to the surface of the chip under a suitable condition is further comprised.

10. The chip surface linker according to claim 9, wherein the suitable condition comprises placing in a drying device at 60°C to 100°C for 1 to 6 h, preferably placing in a drying device at 90°C for 1 h.

11. The chip surface linker according to any one of claims 1 to 10, wherein the chip is a semiconductor chip.

12. The chip surface linker according to claim 11, wherein the surface of the semiconductor chip comprises TiN or TiW, preferably TiN.

13. A method for preparing a chip surface linker, comprising following steps:
step 1: mixing a silanized molecule with a suitable solvent in a volume ratio to obtain a mixed solution;
step 2: contacting and reacting a chip with the mixed solution in the step 1 to form a bonding molecule linked to a surface of the chip; and
step 3: contacting and reacting the surface of the chip linked to the bonding molecule after the reaction with a functional molecule to modify with a hydroxyl group and an ester group.

14. The method according to claim 13, wherein the silanized molecule is selected from one or more of 3-aminopropyltriethoxysilane, dodecyltrimethoxysilane, vinyltriethoxysilane, bis(γ-trimethylsilylpropyl)amine, or γ-(2,3-epoxypropoxy)propyltrimethoxysilane, and is preferably 3-aminopropyltriethoxysilane.

15. The method according to claim 13 or 14, wherein the solvent in the step 1 is selected from one or more of toluene, ethanol or methanol, and is preferably toluene.

16. The method according to any one of claims 13 to 15, wherein a volume ratio of the solvent to the silanized molecule is 2:1 to 40:1, preferably 30:1.

17. The method according to any one of claims 13 to 16, wherein in the step 2, the surface of the chip is contacted and reacted with the mixed solution at 50°C to 100°C for 1 to 8 h, preferably 65°C for 4 h.

18. The method according to any one of claims 13 to 17, wherein the functional molecule in the step 3 is a hydroxyl-containing compound containing a long carbon chain and an ester group.

19. The method according to claim 18, wherein the functional molecule is selected from one or more of a succinic anhydride-modified base monomer, hydroxyethyl methacrylate, a succinic acid-modified base monomer, or an oxalic acid-modified base monomer, and is preferably a succinic anhydride-modified base monomer, wherein the base monomer moiety is selected from one or more of an adenine nucleoside, a guanine nucleoside, a cytosine nucleoside, a thymine nucleoside, or an uracil nucleoside.

20. The method according to any one of claims 13 to 19, wherein before the step 3, the method further comprises a step of curing the bonding molecule linked to the surface of the chip under a suitable condition.

21. The method according to claim 20, wherein the suitable condition comprises placing in a drying device at 60°C to 100°C for 1 to 6 h, preferably placing in a drying device at 90°C for 1 h.

22. The method according to any one of claims 13 to 21, wherein the chip is a semiconductor chip.

23. The method according to claim 22, wherein the surface of the semiconductor chip comprises TiN or TiW, preferably TiN.

24. Use of the chip surface linker according to any one of claims 1 to 12 for nucleic acid synthesis or preparation of chip kits.
